# EUROPÄISCHE PATENTSCHRIFT

(11) **EP 0 999 206 B1**
(45) Veröffentlichungstag und Bekanntmachung des Hinweises auf die Patenterteilung: **18.09.2002**
(21) Anmeldenummer: 99121705.0
(22) Anmeldetag: 02.11.1999
(51) Int. Cl.: C07C 253/14, C07C 255/17

(54) **Verfahren zur Herstellung von alpha-Cyano Essigsäureestern**
Process for producing alpha cyano acetic acid esters
Procéder pour la préparation des esters d'acide acétique alpha cyano

(30) Priorität: 03.11.1998 DE 19850624
(43) Veröffentlichungstag der Anmeldung: 10.05.2000
(73) Patentinhaber: BASF AKTIENGESELLSCHAFT, 67056 Ludwigshafen (DE)
(72) Erfinder: Julius, Manfred, Dr., 67117 Limburgerhof (DE); Schneider, Rolf, Dr., 68163 Mannheim (DE); Mundinger, Klaus, Dr., 67117 Limburgerhof (DE); Fischer, Jakob, Dr., 85414 Kirchdorf (DE)

(56) Entgegenhaltungen:
- EP-A- 0 032 078
- DE-A- 1 951 032
- DE-B- 1 272 914
- US-A- 2 985 682

## Beschreibung

Die vorliegende Erfindung betrifft ein Verfahren zur Herstellung von Cyanessigsäureestern durch Umsetzung eines entsprechenden Monochloressigsäureesters mit Cyanwasserstoff in Gegenwart einer Base.

Cyanessigsäureester sind wichtige Zwischenprodukte zur Herstellung von Effektstoffen, Wirkstoffen (z. B. Coffein) und Lichtschutzmitteln (siehe z. B. WO-A-96 38409) und werden im allgemeinen in zwei Varianten ausgehend von Monochloressigsäure hergestellt:
a) 1. Überführung von Monochloressigsäure in das entsprechende Natriumsalz (z. B. durch Umsetzung mit Natronlauge). 2. Cyanidierung des Natriumsalzes der Monochloressigsäure zum Natriumsalz der Cyanessigsäure (z. B. durch Umsetzung mit NaCN). 3. Freisetzung der Cyanessigsäure und 4. Veresterung der Cyanessigsäure.
b) 1. Veresterung von Monochloressigsäure und 2. Cyanidierung des Monochloressigsäureesters nach folgendem Schema.

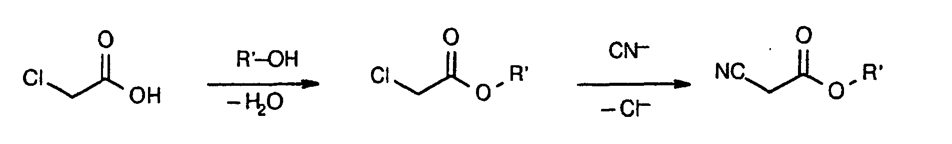

Die Variante b) ist kürzer und aufgrund der geringeren Anzahl an Verfahrensstufen wirtschaftlicher als Variante a). Zudem ist die Menge an anfallenden Salzen, die aufwendig entsorgt werden müssen, geringer.

Die Veresterung von Monochloressigsäure durch Umsetzung mit einem Alkohol ist literaturbekannt, bzw. gelingt in Analogie zu den dem Fachmann bekannten Verfahren. (Siehe z. B.: J. Adhesion Sci. Technol., Vol. 4, No. 9, Seite 734 (1990) und Beispiel Nr. 15).

DE-A-12 10 789 bzw. DE-A-12 72 914 offenbaren die Umsetzung von Monochloressigsäureestern mit Cyanwasserstoff und Alkalimetallcyanid bzw. Alkalimetallalkoholat im molaren Verhältnis von 2 : 1 : 1 bei Temperaturen von ca. 60 °C und Reaktionszeiten von ca. 1 h. Bei einem Monochloressigsäureester-Umsatz von ca. 50 % werden Selektivitäten für die Bildung des entsprechenden Cyanessigsäureesters von ca. 85 % beschrieben.

Gemäß DE-A-19 51 032 werden für die Umsetzung von Monochloressigsäureestern mit überschüssigem NaCN in wäßrigem Acetonitril bei Reaktionstemperaturen von 50 bis 80 °C und Reaktionsdauern von 4 bis 7 h Cyanessigsäureester-Ausbeuten von bis zu 80 % erzielt.

EP-A-32 078 beschreibt die Umsetzung von Monochloressigsäureestern mit HCN und NaCN im Molverhältnis von ca. 1 : 1,2 : 1,6 in wasserfreiem Acetonitril in Gegenwart von tert. Alkoxyalkylaminen als Katalysator. Die Cyanessigsäureester-Ausbeuten liegen nach diesem Verfahren bei Reaktionstemperaturen von 0 bis 20 °C und Reaktionsdauern von 5 bis 10 h bei 93 bis 95 %.

Nachteilig an diesen Verfahren ist das notwendige Handling von festem Natriumcyanid und die aufwendige Rückgewinnung von im Überschuß eingesetzten festen Cyanid-Verbindungen. Ferner muß zunächst in aufwendiger Weise das benötigte Natriumcyanid aus Cyanwasserstoff und Natronlauge hergestellt werden.

US-A-2,985,682 beschreibt ein Verfahren zur Herstellung von Cyanessigsäureestern durch Umsetzung von Monohalogenessigsäureestern mit HCN und Ammoniak. Gemäß Beispiel 1 dieser Patentschrift wird bei der Herstellung von Cyanessigsäure-n-butylester bei einer Reaktionszeit von mindestens 7 h eine Ausbeute von 50 % und eine Selektivität (bezogen auf den eingesetzten Monochloressigsäuren-butylester) von 89 % erzielt.
Nachteilig an diesem Verfahren ist die geringe Raum-Zeit-Ausbeute.

Der vorliegenden Erfindung lag die Aufgabe zugrunde, ein alternatives wirtschaftliches Verfahren zur Herstellung von Cyanessigsäureestern ausgehend von entsprechenden Monochloressigsäureestern durch deren Umsetzung mit Cyanwasserstoff aufzufinden.

Demgemäß wurde ein Verfahren zur Herstellung von Cyanessigsäureestern durch Umsetzung eines entsprechenden Monochloressigsäureesters mit Cyanwasserstoff in Gegenwart einer Base gefunden, welches dadurch gekennzeichnet ist, dass es sich bei der Base um eine Verbindung, ausgewählt aus der Gruppe der tertiären Alkylamine, Salze der Kohlensäure, Salze des Kohlensäurehalbesters, Salze von Carbonsäuren, Amidine, Guanidine und aromatischen N-Heterocyclen, handelt.

Beispiele für erfindungsgemäß als Base einsetzbare tertiäre Alkylamine sind:
Trimethylamin, Triethylamin, Ethyldimethylamin, Tri-n-propylamin, Tri-iso-propylamin, Diethyl-iso-propylamin, Ethyl-di-isopropylamin, Tri-n-butylamin, Tri-n-hexylamin, Tri-n-octylamin, Tri-(2-ethyl-hexyl)amin, N,N,N',N'-Tetramethyl-ethylendiamin, Cyclopentyldimethylamin, Cyclopentyldiethylamin, Cyclohexyldimethylamin, Cyclohexyldiethylamin, Ethyl-di-cyclohexylamin, N-Methyl-pyrrolidin, N-Ethyl-pyrrolidin, N-Methyl-piperidin, N-Ethylpiperidin, N-Methyl-hexamethylenimin, N,N'-Dimethyl-piperazin, N,N'-Diethyl-piperazin, 1,4-Diazabicyclo[2.2.2]octan, N-Methylmorpholin, N-Ethyl-morpholin, N,N-Dimethyl-anilin, N,N-Diethylanilin und 4-Dimethylamino-pyridin. Bevorzugt sind Trialkylamine.

Beispiele für erfindungsgemäß als Base einsetzbare Salze der Kohlensäure sind:
Metallcarbonate, bevorzugt Alkalimetallcarbonate, wie Lithium-, Natrium- und Kaliumcarbonat,
Metallhydrogencarbonate, bevorzugt Alkalimetallhydrogencarbonate, wie Lithium-, Natrium- und Kaliumhydrogencarbonat,
quartäre Ammoniumcarbonate, z. B. Tetraalkylammoniumcarbonate, wie Bis-(Tetramethylammonium)-carbonat, Bis-(Tetraethylammonium)-carbonat, Bis-(Tetra-n-butylammonium)-carbonat, Bis-(Methyl-triethyl-ammonium)-carbonat, Bis-(Benzyl-trimethyl-ammonium)-carbonat, Bis-(2-Hydroxyethyl-trimethyl-ammonium)-carbonat oder mit Carbonat-Ionen beladene basische Anionenaustauscher des Typs I (-CH₂-N(CH₃)₃⁺ - Gruppen enthaltend) oder des Typs II (-CH₂-N⁺(CH₃)₂-CH₂CH₂OH - Gruppen enthaltend),
quartäre Ammoniumhydrogencarbonate, z. B. Tetraalkylammoniumhydrogencarbonate wie sie in EP-A-671 384 beschrieben wurden, wie Tetramethylammonium-hydrogencarbonat, Tetraethylammonium-hydrogencarbonat, Tetra-n-butylammoniumhydrogencarbonat, Benzyl-trimethyl-ammonium-hydrogencarbonat, Methyl-tributyl-ammonium-hydrogencarbonat, Methyl-tridodecyl-ammonium-hydrogencarbonat, Methyltriethyl-ammonium-hydrogencarbonat, Ethyl-tributyl-ammonium-hydrogencarbonat, Phenyl-trimethyl-ammonium-hydrogencarbonat, Phenyl-dimethyl-ethyl-ammonium-hydrogencarbonat, Tris-(2-hydroxyethyl)-methyl-ammonium-hydrogencarbonat, 2-Hydroxyethyl-trimethyl-ammonium-hydrogencarbonat, Cholin-hydrogencarbonat oder mit Hydrogencarbonat-Ionen beladene basische Anionenaustauscher des Typs I (-CH₂-N(CH₃)₃⁺ - Gruppen enthaltend) oder des Typs II (-CH₂-N⁺(CH₃)₂-CH₂CH₂OH- Gruppen enthaltend),
quartäre Phosphoniumcarbonate, z. B. Tetraalkylphosphoniumcarbonate, wie Bis-(Tetramethylphosphonium)-carbonat, Bis-(Tetraethylphosphonium)-carbonat und Bis-(Benzyl-trimethyl-phosphonium)-carbonat, und
quartäre Phosphoniumhydrogencarbonate, z. B. Tetraalkylphosphoniumhydrogencarbonate, wie Tetramethylphosphonium-hydrogencarbonat, Tetraethylphosphonium-hydrogencarbonat, Tetra-n-butylphosphoniumhydrogencarbonat, Benzyl-trimethyl-phosphonium-hydrogencarbonat, Methyl-tributyl-phosphonium-hydrogencarbonat, Methyl-tridodecylphosphonium-hydrogencarbonat, Methyl-triethyl-phosphonium-hydrogencarbonat, Ethyl-tributyl-phosphonium-hydrogencarbonat, Phenyltrimethyl-phosphonium-hydrogencarbonat, Phenyl-dimethyl-ethylphosphonium-hydrogencarbonat, Tris-(2-hydroxyethyl)-methyl-phosphonium-hydrogencarbonat und 2-Hydroxyethyl-trimethyl-phosphonium-hydrogencarbonat.

Beispiele für erfindungsgemäß als Base einsetzbare Salze der Kohlensäurehalbester sind:
Metallalkylcarbonate, bevorzugt Alkalimetallalkylcarbonate, wie Lithium-, Natrium- und Kaliummethylcarbonat, Lithium-, Natriumund Kaliumethylcarbonat,
quartäre Ammoniumalkylcarbonate, z. B. Tetraalkylammonium-alkylcarbonate wie sie in EP-A-671 384 beschrieben wurden, wie Tetramethylammonium-methylcarbonat, Methyl-tri-n-butylammonium-methylcarbonat, Tetraethylammonium-methylcarbonat, Benzyl-trimethyl-ammonium-methylcarbonat, Methyl-tridodecylammonium-methylcarbonat, Tetra-n-butylammonium-methylcarbonat, Tetra-n-butylammonium-methylcarbonat, Methyl-triethyl-ammonium-methylcarbonat, Phenyltrimethyl-ammonium-methylcarbonat, Phenyl-dimethyl-ethyl-ammonium-methylcarbonat, Tris-(2-hydroxyethyl)-methyl-ammonium-methylcarbonat, 2-Hydroxyethyl-trimethyl-ammonium-methylcarbonat oder mit Alkylcarbonat-Ionen, z. B. Methylcarbonat-Ionen, beladene basische Anionenaustauscher des Typs I (-CH₂-N(CH₃)₃⁺ - Gruppen enthaltend) oder des Typs II (-CH₂-N⁺(CH₃)₂-CH₂CH₂OH - Gruppen enthaltend), und
quartäre Phosphoniumalkylcarbonate, z. B. Tetraalkylphosphoniumalkylcarbonate, wie Tri-n-butyl-methyl-phosphonium-methylcarbonat, Triethyl-methyl-phosphonium-methylcarbonat und Triphenyl-methyl-phosphonium-methylcarbonat.

Beispiele für erfindungsgemäß als Base einsetzbare Salze von Carbonsäuren sind:
Metallcarboxylate, bevorzugt Metallcarboxylate von aliphatischen Carbonsäuren, wie Ameisensäure, Essigsäure, Propionsäure, 2-Ethylhexansäure, Adipinsäure. Besonders bevorzugt sind Alkalimetallcarboxylate, wie Lithium-, Natrium- und Kaliumacetat, Natriumpropionat.
Weiterhin bevorzugt sind quartäre Ammoniumcarboxylate, bevorzugt quartäre Ammoniumcarboxylate von aliphatischen Carbonsäuren, wie z. B. oben genannt, z. B. Tetraalkylammoniumcarboxylate, wie Tetramethylammoniumacetat, Tetramethylammoniumpropionat, Tetraethylammoniumacetat und Benzyl-trimethyl-ammoniumacetat.
Der Begriff 'Salze von Carbonsäuren' umfasst hier ebenfalls die sog. 'inneren Salze' (Betaine) der Carbonsäuren, wie quartäre Ammonium-Verbindungen von Aminosäuren, z. B. Trimethylammonioacetat und 1,3-Dimethyl-imidazolium-4-carboxylat.

Beispiele für erfindungsgemäß als Base einsetzbare Amidine sind:
Peralkylierte Amidine, wie Essigsäure-methylamid-methylimid, Benzoesäure-methylamid-methylimid, Essigsäure-methylamid-phenylimid und bicyclische Amidine, wie 1,5-Diazabicyclo[4.3.0]nonen-5 (DBN), 1,8-Diazabicyclo[5.4.0]undecen-7 (DBU), 1,6-Diazabicyclo[5.5.0]dodecen-6, 1,7-Diazabicyclo[6.5.0]tridecen-7, 1,8-Diazabicyclo[7.4.0]tridecen-8, 1,8-Diazabicyclo[7.5.0]tetradecen-8, 1,5-Diazabicyclo[4.4.0]decen-5 (DBD), 1,8-Diazabicyclo[5.3.0]decen-7, 1,10-Diazabicyclo[7.3.0]dodecen-9, 1,10-Diazabicyclo[7.4.0]tridecen-9, 2-Methyl-1,5-diazabicyclo[4.3.0]nonen-5, 3-Methyl-1,5-diazabicyclo[4.3.0]nonen-5, 7-Methyl-1,5-diazabicyclo[4.3.0]nonen-5, 7-Benzyl-1,5-diazabicyclo[4.3.0]nonen-5, 11-Methyl-1,8-diazabicyclo[5.4.0]undecen-7, 10-Methyl-1,8-diazabicyclo[5.4.0]undecen-7, 6-Methyl-1,8-diazabicyclo[5.4.0]undecen-7, 6-Benzyl-1,8-Diazabicyclo[5.4.0]undecen-7, 2-Methyl-1,5-diazabicyclo[4.4.0]decen-5, 3-Methyl-1,5-diazabicyclo[4.4.0]decen-5, 7-Methyl-1,5-diazabicyclo[4.4.0]decen-5 und 7-Benzyl-1,5-diazabicyclo[4.4.0]decen-5. Bevorzugt sind DBN, DBD und DBU, besonders bevorzugt sind DBU und DBN.

Beispiele für erfindungsgemäß als Base einsetzbare Guanidine sind:
Peralkylierte Guanidine, wie Pentamethylguanidin, N-Ethyl-N,N',N',N''-tetramethyl-guanidin, N''-Ethyl-N,N,N',N'tetramethyl-guanidin, N,N-Diethyl-N',N',N''-trimethyl-guanidin, N,N''-Diethyl-N,N',N'-trimethyl-guanidin, Pentaethylguanidin, 2-Phenyl-1,1,3,3-tetramethyl-guanidin, 2-Isopropyl-1,1,3,3-tetramethyl-guanidin, 2-Tert.-butyl-1,1,3,3-tetramethyl-guanidin, Pentaphenyl-guanidin, 2-Methyl-1,1,3,3-tetrabutylguanidin, 2-Phenyl-1,1,3,3-tetrabutylguanidin, 1,1,3,3-Tetramethyl-2-octyl-guanidin, 2-Decyl-1,1,3,3-tetramethylguanidin, Pentaisopropyl-guanidin und 2-Tert.-butyl-1,1,3,3-tetraisopropylguanidin.

Beispiele für erfindungsgemäß als Base einsetzbare aromatische N-Heterocyclen sind:
Pyridin, 2-Methyl-pyridin, 3-Methyl-pyridin, 4-Methyl-pyridin, 4-tert.-Butyl-pyridin, 2,6-Dimethylpyridin, 4,4'-Bipyridyl, 2,2'-Bipyridyl, Chinolin, Isochinolin, Pyrazin, Pyrimidin, s-Triazin, N-Methyl-imidazol und N-Methyl-pyrrol.

Quartäre Ammoniumhydrogencarbonate und -carbonate lassen sich zum Beispiel *in situ* gemäß der EP-A-502 707 durch die Umsetzung von entsprechenden Tetraalkylammoniumhalogeniden mit Alklimetallhydrogencarbonaten bzw. Alklimetallcarbonaten herstellen.

Die Herstellung von mit Hydrogencarbonat-Ionen beladenen basischen Ionenaustauschern ist z. B. in WO 95/20559 beschrieben.

Quartäre Phosphoniumhydrogencarbonate und -carbonate lassen sich zum Beispiel *in situ* gemäß der EP-A-502 707 durch die Umsetzung von entsprechenden Tetraalkylphosphoniumhalogeniden mit Alklimetallhydrogencarbonaten bzw. Alklimetallcarbonaten herstellen.

Quartäre Ammonium- bzw. Phosphoniumalkylcarbonate können nach EP-A-291 074 und den dort zitierten Methoden, zum Beispiel durch Umsetzung eines tertiären Amins bzw. Phosphins mit einem Dialkylcarbonat in etwa stöchiometrischen Mengen, hergestellt werden. Die dabei entstehende Lösung kann direkt ohne einen weiteren Verfahrensschritt im erfindungsgemäßen Verfahren als Base eingesetzt werden.

Die Synthese von peralkylierten Amidinen ist z. B. in Houben-Weyl, Methoden der organischen Chemie, Band E5, S. 1304-8 (1985); S. Patai, The chemistry of amidines and imidates, S. 283f (1975); H. Oediger et al., Synthesis, S. 591-8 (1972); H. Oediger et al., Chem. Ber. 99, S. 2012-16 (1966).; L. Xing-Quan, J. Nat. Gas Chem. 4, S. 119-27 (1995) und der älteren deutschen Patentanmeldung Nr. 19752935.6 beschrieben.

Die Synthese von peralkylierten Guanidinen ist z. B. in US-A-2,845,459; Justus Liebigs Ann. Chem. 445, S. 70 (1925); ibd. 438, S. 163 (1924); ibd. 455, S. 163f (1927); ibd. 455, S. 152 (1927); Chem. Ber. 97, 1232-45 (1964); Tetrahedron 26, 1805-20 (1979); Chem. Ber. 37, 965 (1904); Tetrahedron 46 (6), 1839-48 (1990); Liebigs Ann. Chem., 108-26 (1984); ibd. 2178-93 (1985); FR-A-25 09 724; US-A-4,358,613; GB 12 90 470 und US-A-3,399,233 beschrieben.

Die Herstellung des Betains 1,3-Dimethyl-imidazolium-4-carboxylat durch Umsetzung von 1-Methyl-imidazol mit Dimethylcarbonat ist unten im Beispiel Nr. 16 beschrieben (vgl. ältere deutschen Patentanmeldung Nr. 19836477.6).

### Cyanessigsäureester der Formel I

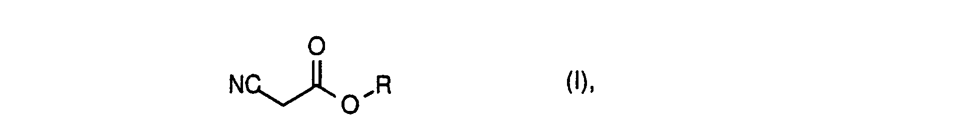

in welcher R einen gesättigten oder ungesättigten aliphatischen, cycloaliphatischen oder heterocyclischen Rest, aromatischen oder heteroaromatischen Rest oder Arylalkylrest bedeutet, wobei der Rest R unter den Reaktionsbedingungen inerte Substituenten tragen kann, sind wirtschaftlich von besonderem Interesse.
Das erfindungsgemäße Verfahren findet daher bevorzugt zur Herstellung der Cyanessigsäureester I Anwendung, indem man einen Monochloressigsäureester der Formel II 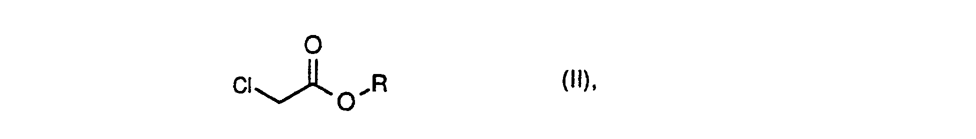 mit Cyanwasserstoff umsetzt.
Der Rest R ist breit variierbar, beispielsweise seien genannt:
- linearer oder verzweigter gesättigter aliphatischer Rest, bevorzugt C₁₋₂₀-Alkyl, besonders bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neoPentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl,
   ganz besonders bevorzugt C₁₋₈-Alkyl, wie Methyl, Ethyl, n-Propyl, iso-Propyl, n-Butyl, iso-Butyl, sec.-Butyl, tert.-Butyl und 2-Ethyl-hexyl.
- linearer oder verzweigter, ein- oder mehrfach ungesättigter aliphatischer Rest, bevorzugt C₂₋₂₀-Alkenyl und C₃₋₂₀-Alkinyl, besonders bevorzugt C₂₋₁₂-Alkenyl und C₃₋₁₂-Alkinyl, wie Ethenyl, 2-Propen-1-yl, 2-Propen-2-yl, 2-Butenl-yl, 2-Buten-2-yl, 3-Buten-1-yl, 3-Buten-2-yl, 2-Penten-1-yl, 4-Penten-1-yl, 2-Hexen-1-yl, 5-Hexen-1-yl, 2-Propin-1-yl, 2-Butin-1-yl, 3-Butin-1-yl, 1-Butin-3-yl, 1-Butin-3-methyl-3-yl, 2-Pentin-1-yl, 4-Pentin-1-yl, 2-Hexin-1-yl, 5-Hexin-1-yl, 1-Pentin-3-methyl-3-yl, 1-Octin-4-ethyl-3-yl,
- gesättigter cycloaliphatischer Rest, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl, ganz besonders bevorzugt Cyclopentyl und Cyclohexyl,
- ungesättigter cycloaliphatischer Rest, bevorzugt C₅₋₁₂-Cycloalkenyl, besonders bevorzugt C₅₋₈-Cycloalkenyl, wie 1-Cyclopentenyl, 3-Cyclopentenyl, 1-Cyclohexenyl, 3-Cyclohexenyl, 1-Cycloheptenyl und 1-Cyclooctenyl,
- heterocyclischer Rest, bevorzugt C₃₋₁₅-Heterocycloalkyl, wie N-Alkyl-piperidin-3-yl, N-Alkyl-piperidin-4-yl, N,N'-Dialkylpiperazin-2-yl, Tetrahydrofuran-3-yl, N-Alkyl-pyrrolidin-3-yl.
- aromatischer Rest, bevorzugt C₆₋₂₀-Aryl, wie Phenyl, 1-Naphthyl, 2-Naphthyl, 1-Anthryl, 2-Anthryl, 9-Anthryl, besonders bevorzugt Phenyl, 1-Naphthyl und 2-Naphthyl, besonders bevorzugt Phenyl,
- heteroaromatischer Rest, bevorzugt C₃₋₁₅-Heteroaryl, wie 2-Pyridinyl, 3-Pyridinyl, 4-Pyridinyl, Chinolinyl, Pyrazinyl, Pyrrol-3-yl, Thienyl, Imidazol-2-yl, 2-Furanyl und 3-Furanyl,
- Arylalkylrest, bevorzugt C₇₋₂₀-Arylalkyl, wie Benzyl, 1-Phenethyl, 2-Phenethyl, 1-Naphthylmethyl, 2-Naphthylmethyl, Phenanthrylmethyle, 4-tert.-Butyl-phenyl-methyl, 1-Phenylpropyl, 2-Phenylpropyl, 3-Phenylpropyl, 1-Phenylbutyl, 2-Phenylbutyl, 3-Phenylbutyl und 4-Phenylbutyl,
   besonders bevorzugt Benzyl, 1-Phenethyl und 2-Phenethyl,
wobei in diesen Fällen der Rest R unter den Reaktionsbedingungen inerte Substituenten tragen kann, wie z. B. C₁₋₂₀-Alkyl, C₁₋₂₀-Alkoxy, C₆₋₂₀-Aryloxy und Halogen. Dabei kann die Anzahl dieser Substituenten in R in Abhängigkeit von der Art des Restes 0 bis 5, vorzugsweise 0 bis 3, insbesondere 0, 1 oder 2 betragen. Als Substituenten kommen in Betracht:
- C₁₋₂₀-Alkyl, wie oben definiert,
- C₁₋₂₀-Alkoxy, bevorzugt C₁₋₈-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁₋₄-Alkoxy, wie Methoxy, Ethoxy, n-Propoxy, iso-Propoxy, n-Butoxy, iso-Butoxy, sec.-Butoxy und tert.-Butoxy,
- C₆₋₂₀-Aryloxy, wie Phenoxy, 1-Naphthoxy und 2-Naphthoxy, bevorzugt Phenoxy,
- Halogen, wie Fluor, Chlor, Brom.

Das erfindungsgemäße Verfahren zur Herstellung von Cyanessigsäureestern lässt sich wie folgt ausführen:
Man kann beispielsweise
   a) eine Mischung des entsprechenden Monochloressigsäureesters mit Cyanwasserstoff, gegebenenfalls in Gegenwart eines inerten Lösungsmittels, vorlegen und anschließend die Base, gegebenenfalls in einem inerten Lösungsmittel, zudosieren oder
   b) eine Mischung der Base mit Cyanwasserstoff, gegebenenfalls in Gegenwart eines inerten Lösungsmittels, vorlegen und anschließend den entsprechenden Monochloressigsäureester, gegebenenfalls in einem inerten Lösungsmittel, zudosieren oder
   c) eine Mischung der Base mit dem entsprechenden Monochloressigsäureester, gegebenenfalls in Gegenwart eines inerten Lösungsmittels, vorlegen und anschließend Cyanwasserstoff, gegebenenfalls in einem inerten Lösungsmittel, zudosieren.
   d) die Base in Gegenwart eines inerten Lösungsmittels vorlegen und anschließend eine Mischung des entsprechenden Monochloressigsäureesters mit Cyanwasserstoff, gegebenenfalls in einem inerten Lösungsmittel, zudosieren.

Die Varianten a), b) und d) sind hierbei bevorzugt.

Das Verfahren kann bei Reaktionstemperaturen von -78 bis 200 °C, bevorzugt -30 bis 100 °C, besonders bevorzugt -25 bis 80 °C, ganz besonders bevorzugt 0 bis 60 °C, durchgeführt werden.

Der Reaktionsdruck (absolut gemessen) beträgt in der Regel 0,05 bis 2 MPa (0,5 bis 20 bar), bevorzugt 0,09 bis 1 MPa (0,9 bis 10 bar), besonders bevorzugt Atmosphärendruck (Normaldruck).

Die Base wird in der Regel in Mengen von 50 bis 300 Mol-%, bevorzugt 75 bis 150 Mol-%, besonders bevorzugt 95 bis 105 Mol-%, ganz besonders bevorzugt 100 Mol-%, bezogen auf den eingesetzten Monochloressigsäureester, eingesetzt.

Im erfindungsgemäßen Verfahren wird im allgemeinen der Cyanwasserstoff in Mengen von 50 bis 800 Mol-% bezogen auf den Monochloressigsäureester eingesetzt. Auch höhere Cyanwasserstoff-Überschüsse sind möglich.
Das molare Verhältnis der beiden Einsatzstoffe Cyanwasserstoff und Monochloressigsäureester beträgt in der Regel 0,75 bis 6 : 1, bevorzugt 0,9 bis 5 : 1, besonders bevorzugt 1 bis 4 : 1, ganz besonders bevorzugt 2 bis 4 : 1.
Evtl. nicht umgesetzter sowie der im Überschuss eingesetzte Cyanwasserstoff kann aus dem Reaktionsrohprodukt destillativ zurückgewonnen und wieder zurückgeführt werden.

Mit den erfindungsgemäß verwendeten Basen sind besonders kurze Verweilzeiten des Reaktionsgemisches im Reaktor (= Reaktionszeit) bei guten bis sehr guten Ausbeuten, Selektivitäten und hohen Raum-Zeit-Ausbeuten erzielbar. Je nach den gewählten Reaktionsbedingungen betragen die Verweilzeiten im allgemeinen 10 Minuten bis wenige Stunden, bevorzugt 0,5 bis 5 Stunden, besonders bevorzugt 0,5 bis 3 Stunden.

Weiterhin wird im erfindungsgemäßen Verfahren die unter basischen Bedingungen als Nebenreaktion bekannte 'Dimerisierung' und 'Trimerisierung' des Monochloressigsäureesters zu den ungewünschten Estern III und IV 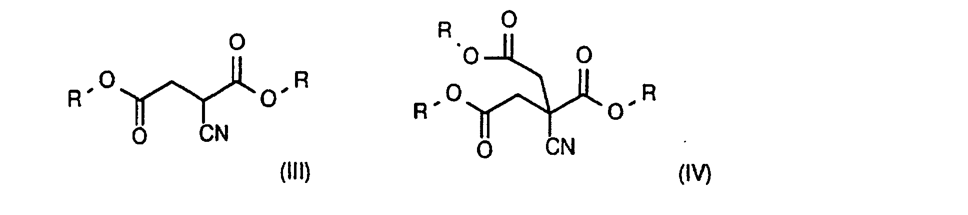 nahezu vollständig vermieden und zwar im besonderen dann, wenn der Cyanwasserstoff im molaren Überschuss bezogen auf den Monochloressigsäureester eingesetzt wird.

Das erfindungsgemäße Verfahren kann auch kontinuierlich durchgeführt werden. Beispielsweise kann eine kontinuierliche Verfahrensdurchführung derart erfolgen, dass die Base, gegebenenfalls zusammen mit einem inerten Lösungsmittel, kontinuierlich in einen Reaktor gespeist wird, in dem kontinuierlich eine Mischung des entsprechenden Monochloressigsäureesters mit Cyanwasserstoff, gegebenenfalls in Gegenwart eines Lösungsmittels, umgesetzt wird.

Als Reaktionsgefäße bzw. Reaktoren für das erfindungsgemäße Verfahren eignen sich beispielsweise Rührreaktoren, Rohrreaktoren, Rührbehälterkaskaden oder Mischkreise.

Das erfindungsgemäße Verfahren wird bevorzugt in Abwesenheit von Wasser durchgeführt.

In manchen Fällen hat es sich als vorteilhaft erwiesen, die erfindungsgemäße Umsetzung in Gegenwart von 0,5 bis 10 Mol-%, bevorzugt 1 bis 5 Mol-%, bezogen auf den Monochloressigsäureester, eines Katalysators, ausgewählt aus der Gruppe der Bis-(dialkylaminoalkyl)ether, wie z. B. Bis-(2-dimethylaminoethyl)ether, oder der Tris-(alkoxyalkyl)amine, wie z. B. Tris-(methoxy-ethyl)amin (= (MeOCH₂CH₂)₃N), Tris-(ethoxy-ethyl)amin, Tris- (methoxyethoxyethyl)amin (= TDA-1) 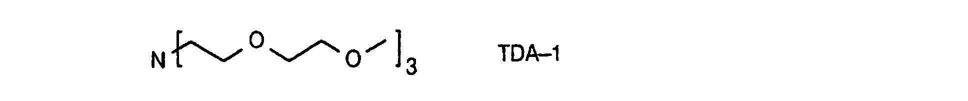 oder Tris-(ethoxyethoxyethyl)amin (= TDA-2), durchzuführen.

Das erfindungsgemäße Verfahren kann in Gegenwart oder Abwesenheit von inerten Lösungsmitteln vorgenommen werden.

Als inerte Lösungsmittel eignen sich Ether, wie Diethylether, Methyl-tert.-butyl-ether, Tetrahydrofuran und Dioxan, Nitrile, wie Acetonitril und Propionitril, aliphatische Kohlenwasserstoffe, wie n-Pentan, Pentanisomerengemische, n-Hexan, Hexanisomerengemische, n-Heptan, Heptanisomerengemische, n-Octan, Octanisomerengemische, cycloaliphatische Kohlenwasserstoffe, wie Cyclopentan, Cyclohexan, Cycloheptan und Cyclooctan, Alkohole, bevorzugt C₁bis C₄-Alkanole, wie Methanol, Ethanol, n-Propanol, iso-Propanol, n-Butanol, iso-Butanol und tert.-Butanol, Amide, wie Dimethylformamid, Dimethylacetamid, N-Methylpyrrolidon, Harnstoffe, wie N,N'-Dimethylethylenharnstoff, N,N'-Dimethylpropylenharnstoff, N,N,N',N'-Tetra-n-butylharnstoff, Carbonate, wie Ethylencarbonat und Propylencarbonat, Sulfolan, Dimethylsulfoxid oder Kohlendioxid in flüssigem oder überkritischem Zustand.

Das erfindungsgemäße Verfahren wird wird bevorzugt in Gegenwart polarer aprotischer Lösungsmittel, wie Dimethylformamid, Dimethylacetamid, Acetonitril und Dimethylsulfoxid durchgeführt.

Bei Verwendung von tertiären Alkylaminen, Amidinen, Guanidinen oder aromatischen N-Heterocyclen als Base kann gegebenenfalls nach der erfolgten Umsetzung das aus dem Reaktionsrohgemisch ausgefallene, oder durch Zusatz eines unpolaren aprotischen Lösungsmittels, wie z. B. Pentan oder Cyclohexan, ausgefällte, Hydrochlorid der Base abgetrennt werden, z. B. durch Filtration.

Die Reingewinnung der Cyanessigsäureester erfolgt in der Regel durch eine fraktionierte Rektifikation des Reaktionsrohproduktes oder durch Kristallisation/Umkristallisation des Cyanessigsäureesters.

Dabei zurückgewonnene unumgesetzte Edukte und Lösungsmittel können wieder in die Synthese eingesetzt werden.

Aus dem gegebenenfalls aus dem Reaktionsaustrag abgetrennten Hydrochlorid der Base, wie z. B. Triethylamin-hydrochlorid, Cyclohexyldimethylamin-hydrochlorid oder Diisopropylethylamin-hydrochlorid, kann nach dem Fachmann bekannten Verfahren die Base wieder freigesetzt und danach erneut im erfindungsgemäßen Verfahren eingesetzt werden.

### Beispiele

Die bei den Beispielen Nr. 1 bis 15 angewandten GC-Bedingungen waren: Säule: 30 m DB-1; 1 µm Filmdicke. Temperaturprogramm: 50 bis 300 °C, 10 °C/Min., 35 Min. 300 °C.

Die Reinheit des in den Beispielen Nr. 1 bis 9 eingesetzten Monochloressigsäure-(2-ethyl-hexyl)esters betrug nach GC größer 99 %. Zur Synthese dieses Esters siehe Beispiel Nr. 15.
Die Reinheit des in den Beispielen Nr. 10 bis 14 eingesetzten Monochloressigsäureethylesters betrug nach GC größer 99 %.

In den Beispielen Nr. 1 bis 14 sind die Angaben für den Umsatz und die Selektivität der Reaktion stets auf den eingesetzten Monochloressigsäure-(2-ethyl-hexyl)ester bzw. Monochloressigsäureethylester bezogen und wurden gaschromatographisch im erhaltenen Reaktionsrohprodukt ermittelt. Dazu wurden die gezogenen Proben aus dem Rohprodukt bis zur gaschromatographischen Analyse in Trockeneis (-78 °C) eingefroren.

### Beispiel 1

20,65 g (100 mmol) Monochloressigsäure-(2-ethyl-hexyl)ester, 2,7 g (100 mmol) frisch destillierter Cyanwasserstoff und 0,97 g (3 mmol) Tris-(dioxa-3,6-heptyl)-amin (= TDA-1) wurden in 70 ml trockenem Acetonitril bei 0 °C unter einer Argon-Atmoshäre vorgelegt. Anschließend wurde unter Rühren während ca. 1 h eine Lösung von 12,7 g (100 mmol) Dimethylcyclohexylamin in 30 ml trockenem Acetonitril zudosiert, wobei die Reaktionsmischung bei einer Temperatur von 0 bis 5 °C gehalten wurde. Die Analyse des erhaltenen Rohprodukts zeigte einen Umsatz von 86,4 % und eine Selektivität für Cyanessigsäure-(2-ethyl-hexyl)ester von 50,3 %.

### Beispiel 2

Eine Durchführung der Umsetzung analog Beispiel 1, mit dem Unterschied, dass die Reaktion bei -20 °C durchgeführt wurde, ergab einen Umsatz von 85,5 % bei einer Selektivität für Cyanessigsäure-(2-ethyl-hexyl)ester von 52,3 %.

### Beispiel 3

20,65 g (100 mmol) Monochloressigsäure-(2-ethyl-hexyl)ester und 2,7 g (100 mmol) frisch destillierter Cyanwasserstoff wurden in 70 ml trockenem Acetonitril bei 20 °C unter einer Argon-Atmoshäre vorgelegt. Anschließend wurde unter Rühren während ca. 1 h eine Lösung von 15,5 g (100 mmol) 1,8-Diazabicyclo-[5.4.0]-undec-7-en (DBU) in 30 ml trockenem Acetonitril zudosiert, wobei die Reaktionsmischung durch Kühlung bei einer Temperatur von 20 °C gehalten wurde. Die Analyse des erhaltenen Rohprodukts zeigte einen Umsatz von 93,6 % und eine Selektivität für Cyanessigsäure-(2-ethyl-hexyl)ester von 47,0 %.

### Beispiel 4

10,3 g (50 mmol) Monochloressigsäure-(2-ethyl-hexyl)ester und 1,3 g (50 mmol) frisch destillierter Cyanwasserstoff wurden in 40 ml trockenem Acetonitril bei 20 °C unter einer Argon-Atmoshäre vorgelegt. Anschließend wurde unter Rühren während ca. 1 h eine Lösung von 5,05 g (50 mmol) Triethylamin in 30 ml trockenem Acetonitril zudosiert, wobei die Reaktionsmischung bei einer Temperatur von 20 °C gehalten wurde. Die Analyse des erhaltenen Rohprodukts zeigte einen Umsatz von 54,1 % und eine Selektivität für Cyanessigsäure-(2-ethyl-hexyl)-ester von 68,0 %.
Nach einer Nachreaktionszeit von 4 h bei 20 °C betrug der Umsatz 61,9 % bei einer Selektivität für Cyanessigsäure-(2-ethyl-hexyl)ester von 72,2 %.

### Beispiel 5

Eine Durchführung der Umsetzung analog Beispiel 4, mit dem Unterschied, dass die Reaktion bei 50 °C durchgeführt wurde, ergab einen Umsatz von 57,3 % bei einer Selektivität für Cyanessigsäure-(2-ethyl-hexyl)ester von 66,7 %.
Nach einer Nachreaktionszeit von 4 h bei 50 °C betrug der Umsatz 70,5 % bei einer Selektivität für Cyanessigsäure-(2-ethylhexyl)ester von 68,8 %.

### Beispiel 6

Eine Durchführung der Umsetzung analog Beispiel 4, mit dem Unterschied, dass die Reaktion unter Verwendung von 6,46 g (50 mmol) N-Ethyl-diisopropylamin (Hünig-Base) durchgeführt wurde, ergab einen Umsatz von 33,2 % bei einer Selektivität für Cyanessigsäure-(2-ethyl-hexyl)ester von 73,5 %.
Nach einer Nachreaktionszeit von 5 h bei 20 °C betrug der Umsatz 42,1 % bei einer Selektivität für Cyanessigsäure-(2-ethylhexyl)ester von 74,8 %.

### Beispiel 7

10,3 g (50 mmol) Monochloressigsäure-(2-ethyl-hexyl)ester und 5,61 g (27 mmol) Bis-(Tetramethylammonium)-carbonat) wurden in 40 ml trockenem Acetonitril bei 20 °C unter einer Argon-Atmoshäre vorgelegt. Nach 15 Min. Rühren wurde unter fortgesetztem Rühren während ca. 1 h eine Lösung von 1,3 g (50 mmol) frisch destilliertem Cyanwasserstoff in 40 ml trockenem Acetonitril zudosiert, wobei die Reaktionsmischung durch zeitweise Kühlung bei einer Temperatur von 20 bis 25 °C gehalten wurde. Die Analyse des erhaltenen Rohprodukts zeigte einen Umsatz von 50,3 % und eine Selektivität für Cyanessigsäure-(2-ethyl-hexyl)-ester von 72,4 %.
Nach einer Nachreaktionszeit von 5,5 h bei 20 °C betrug der Umsatz 69,3 % bei einer Selektivität für Cyanessigsäure-(2-ethylhexyl)ester von 78,4 %.

### Beispiel 8

1,3 g (50 mmol) frisch destillierter Cyanwasserstoff und 7,45 g (50 mmol) Tetramethylammoniummethylcarbonat wurden in 40 ml trokkenem Acetonitril bei 20 °C unter einer Argon-Atmoshäre vorgelegt. Anschließend wurde unter Rühren während ca. 1 h eine Lösung von 10,3 g (50 mmol) Monochloressigsäure-(2-ethyl-hexyl)ester in 30 ml trockenem Acetonitril zudosiert, wobei die Reaktionsmischung bei einer Temperatur von 20 °C gehalten wurde und danach 1 h bei 20 °C nachgerührt. Die Analyse des erhaltenen Rohprodukts zeigte einen Umsatz von 91,0 % und eine Selektivität für Cyanessigsäure-(2-ethyl-hexyl)ester von 74,3 %.

### Beispiel 9

1,3 g (50 mmol) frisch destillierter Cyanwasserstoff und 6,4 g (46 mmol) 1,3-Dimethyl-imidazolium-4-carboxylat wurden in 40 ml trockenem Acetonitril bei 20 °C unter einer Argon-Atmoshäre vorgelegt. Anschließend wurde unter Rühren während ca. 1 eine Lösung von 10,3 g (50 mmol) Monochloressigsäure-(2-ethyl-hexyl)ester in 30 ml trockenem Acetonitril zudosiert, wobei die Reaktionsmischung bei einer Temperatur von 24 bis 30 °C gehalten wurde. Die Analyse des erhaltenen Rohprodukts zeigte einen Umsatz von 90,4 % und eine Selektivität für Cyanessigsäure-(2-ethyl-hexyl)ester von 86,7 %.
Nach einer Nachreaktionszeit von 1 h bei 20 °C betrug der Umsatz 93,1 % bei einer Selektivität für Cyanessigsäure-(2-ethylhexyl)ester von 88,0 %.

### Beispiel 10

10,6 g (0,1 Mol) wasserfreies Natriumcarbonat wurden in 50 ml trockenem Dimethylacetamid bei 50 °C unter Rühren suspendiert und während 30 Min. eine Mischung von 12,25 g (0,1 Mol) Monochloressigsäureethylester und 2,7 g (0,1 Mol) destilliertem wasserfreien Cyanwasserstoff zudosiert. Die Reaktion verlief exotherm, sodass sich die Reaktionsmischung kurzzeitig auf 55 °C erwärmte. Es wurde bei 50 °C nachgerührt. Die Analyse des erhaltenen Rohprodukts zeigte nach einer Gesamtreaktionszeit von 3 h einen Umsatz von 75,4 % und eine Selektivität für Cyanessigsäureethylester von 83,5 %.
Nach einer Gesamtreaktionszeit von 4 h betrug der Umsatz 88,1 % bei einer Selektivität für Cyanessigsäureethylester von 77,0 %.

### Beispiel 11

Bei Durchführung des Versuchs wie in Beispiel 10 beschrieben, mit dem Unterschied, dass während 60 Min. eine Mischung von 12,25 g (0,1 Mol) Monochloressigsäureethylester und 5,4 g (0,2 Mol) destilliertem wasserfreien Cyanwasserstoff zudosiert wurde, ergab die Analyse des erhaltenen Rohprodukts nach einer Gesamtreaktionszeit von 1 h einen Umsatz von 63,2 % und eine Selektivität für Cyanessigsäureethylester von 92,0 %.
Nach einer Gesamtreaktionszeit von 2 h betrug der Umsatz 92,1 % bei einer Selektivität für Cyanessigsäureethylester von 91,1 %.

### Beispiel 12

Bei Durchführung des Versuchs wie in Beispiel 10 beschrieben, mit dem Unterschied, dass während 45 Min. eine Mischung von 12,25 g (0,1 Mol) Monochloressigsäureethylester und 8,1 g (0,3 Mol) destilliertem wasserfreien Cyanwasserstoff zudosiert wurde, ergab die Analyse des erhaltenen Rohprodukts nach einer Gesamtreaktionszeit von 45 Min. einen Umsatz von 65,0 % und eine Selektivität für Cyanessigsäureethylester von 95,2 %.
Nach einer Gesamtreaktionszeit von 2 h betrug der Umsatz 96,1 % bei einer Selektivität für Cyanessigsäureethylester von 94,1 %.

### Beispiel 13

Bei Durchführung des Versuchs wie in Beispiel 10 beschrieben, mit dem Unterschied, dass während 60 Min. eine Mischung von 12,25 g (0,1 Mol) Monochloressigsäureethylester und 10,8 g (0,4 Mol) destilliertem wasserfreien Cyanwasserstoff zudosiert wurde, ergab die Analyse des erhaltenen Rohprodukts nach einer Gesamtreaktionszeit von 1 h einen Umsatz von 77,8 % und eine Selektivität für Cyanessigsäureethylester von 96,4 %.
Nach einer Gesamtreaktionszeit von 2 h betrug der Umsatz 97,5 % bei einer Selektivität für Cyanessigsäureethylester von 96,2 %.

### Beispiel 14

Bei Durchführung des Versuchs wie in Beispiel 10 beschrieben, mit dem Unterschied, dass die Reaktion bei 40 °C durchgeführt und während 60 Min. eine Mischung von 12,25 g (0,1 Mol) Monochloressigsäureethylester und 8,1 g (0,3 Mol) destilliertem wasserfreien Cyanwasserstoff zudosiert wurde, ergab die Analyse des erhaltenen Rohprodukts nach einer Gesamtreaktionszeit von 3 h einen Umsatz von 81,9 % und eine Selektivität für Cyanessigsäureethylester von 95,2 %.
Nach einer Gesamtreaktionszeit von 5 h betrug der Umsatz 92,5 % bei einer Selektivität für Cyanessigsäureethylester von 94,0 %.

### Beispiel 15: Synthese von Monochloressigsäure-(2-ethylhexyl)ester

Eine Mischung von 630 g einer 75 %igen wässrigen Lösung von Monochloressigsäure (5,0 Mol), 250 ml Toluol und 5 g konz. Schwefelsäure wurden unter Rühren am Wasserabscheider zum Sieden erhitzt und während 105 Min. 693 g (5,33 Mol) 2-Ethylhexanol-1 hinzugefahren. Das Erhitzen zum Rückfluß wurde danach weitere 6 h fortgesetzt, wobei insgesamt 246 g Wasser (99,4 % d.Th.) abgeschieden wurden. Die Reaktionslösung wurde nach dem Abkühlen mit gesättigter wässriger NaHCO₃-Lösung gewaschen, die organische Phase abgetrennt und in einer Füllkörperkolonne (Länge 50 cm) bei 15 mbar fraktioniert rektifiziert. Bei einer Übergangstemperatur von 117 °C erhielt man 880,6 g Monochloressigsäure-(2-ethylhexyl)ester als farblose Flüssigkeit mit einer Reinheit von 99,43 % (nach GC). Ausbeute: 85 %.

### Beispiel 16: Herstellung von 1,3-Dimethyl-imidazolium-4-carboxylat (Norzooanemonin)

In einem Autoklaven wurden 0,9 mol (73,8 g) 1-Methyl-imidazol und 0,9 mol (81,0 g) Dimethylcarbonat bei Raumtemperatur vorgelegt. Der Ansatz wurde anschließend auf 140 °C erwärmt und 20 Stunden bei dieser Temperatur gerührt (Eigendruck: ca. 0,5 MPa). Nach dem Abkühlen auf Raumtemperatur verblieb eine gelbe dicke Suspension. Die Kristalle wurden abfiltriert und im Ölpumpenvakuum getrocknet. Rohgewicht: 119,4 g (= 94,7 % Rohausbeute).
Die Kristalle wurden aus einem ca. 1/1 Ethanol/Methanol-Gemisch umkristallisiert. Dabei fielen weiße Kristalle an. Diese wurden abfiltriert und im Hochvakuum getrocknet. Gewicht: 48,9 g.

Die Mutterlauge wurde zur Trockene eingeengt. Der verbleibende, gelb-ölige Rückstand wurde erneut in heißem 1/1 Ethanol/Methanol-Gemisch aufgenommen und in der Kälte ausgefällt. Es resultierten weitere 53,9 g Produkt.
Gesamtausbeute: 102,8 g (81,5 %).

Charakterisierung des Produkts:
Schmelzpunkt: 240 °C (Zersetzung).
MS (Elektronenspray Ionisation ESI, Direkteinlaß): M = 141 (M+H)⁺.

| | | |
|---|---|---|
| Elementaranalyse C₆H₈N₂O₂; MG = 140,14 | berechnet | C 51,4; H 5,8; N 20,0; O 22,8 |
| | gefunden | C 51,3; H 5,7; N 19,9; O 23,4. |

¹H-NMR (400 MHz, D₂O) δ (ppm) = 3,98 (3 H); 4,12 (3H); 7,88 (1H); 1 H tauscht in D₂O aus.
¹³C-NMR (100,61 MHz, D₂O) δ (ppm) = 38,55 (Methyl); 38,65 (Methyl); 129,03 (CH); 133,59 (C-COO⁻); 140,82 (t, Kopplung C-D); 165,8 (COO⁻).

IR (KBr), [cm⁻¹]: 3451 ss, 1621 ss.

## Patentansprüche

1. Verfahren zur Herstellung von Cyanessigsäureestern durch Umsetzung eines entsprechenden Monochloressigsäureesters mit Cyanwasserstoff in Gegenwart einer Base, **dadurch gekennzeichnet, dass** es sich bei der Base um eine Verbindung, ausgewählt aus der Gruppe der tertiären Alkylamine, Salze der Kohlensäure, Salze der Kohlensäurehalbester, Salze von Carbonsäuren, Amidine, Guanidine und aromatischen N-Heterocyclen, handelt.

2. Verfahren nach Anspruch 1, **dadurch gekennzeichnet, dass** es sich bei der Base um eine Verbindung, ausgewählt aus der Gruppe der Alkalimetallcarbonate, quartäre Ammoniumcarbonate, quartäre Ammoniumalkylcarbonate, Triethylamin, N-Ethyl-diisopropylamin, Tri-n-butylamin und Dimethylcyclohexylamin, handelt.

3. Verfahren nach Anspruch 1, **dadurch gekennzeichnet, dass** es sich bei der Base um Natriumcarbonat oder 1,3-Dimethyl-imidazolium-4-carboxylat handelt.

4. Verfahren nach den Ansprüchen 1 bis 3, **dadurch gekennzeichnet, dass** das molare Verhältnis von Cyanwasserstoff zu Monochloressigsäureester 1 bis 4 : 1 beträgt.

5. Verfahren nach den Ansprüchen 1 bis 4, **dadurch gekennzeichnet, dass** die Umsetzung bei Temperaturen von -25 bis 80 °C durchgeführt wird.

6. Verfahren nach den Ansprüchen 1 bis 5, **dadurch gekennzeichnet, dass** die Umsetzung in Gegenwart von 75 bis 150 Mol-% der Base, bezogen auf den eingesetzten Monochloressigsäureester, durchgeführt wird.

7. Verfahren nach den Ansprüchen 1 bis 6, **dadurch gekennzeichnet, dass** die Umsetzung in Gegenwart eines Katalysators, ausgewählt aus der Gruppe der Bis-(dialkylaminoalkyl)ether und Tris-(alkoxyalkyl)amine, durchgeführt wird.

## Claims

1. A process for the preparation of cyanoacetic esters by reacting a corresponding monochloroacetic ester with hydrogen cyanide in the presence of a base, wherein the base is a compound selected from the group consisting of tertiary alkylamines, salts of carbonic acid, salts of carbonic acid monoesters, salts of carboxylic acids, amidines, guanidines and aromatic N-heterocyclic compounds.

2. A process as claimed in claim 1, wherein the base is a compound selected from the group consisting of alkali metal carbonates, quaternary ammonium carbonates, quaternary ammonium alkyl carbonates, triethylamine, N-ethyldiisopropylamine, tri-n-butylamine and dimethylcyclohexylamine.

3. A process as claimed in claim 1, wherein the base is sodium carbonate or 1,3-dimethylimidazolium-4-carboxylate.

4. A process as claimed in any one of claims 1 to 3, wherein the molar ratio between hydrogen cyanide and monochloroacetic ester is from 1 to 4 : 1.

5. A process as claimed in any one of claims 1 to 4, wherein the reaction is carried out at temperatures of from -25 to 80°C.

6. A process as claimed in any one of claims 1 to 5, wherein the reaction is carried out in the presence of from 75 to 150 mol% of the base, based on the monochloroacetic ester employed.

7. A process as claimed in any one of claims 1 to 6, wherein the reaction is carried out in the presence of a catalyst selected from the group consisting of bis(dialkylaminoalkyl) ethers and tris(alkoxyalkyl)amines.

## Revendications

1. Procédé pour la préparation des esters d'acide cyano-acétique au moyen d'une réaction d'un ester d'acide mono-chloro-acétique correspondant avec un cyanure en présence d'une base, **caractérisé en ce que** la base est un composé choisi dans le groupe formé par les alkylamines tertiaires, les sels de l'acide carbonique, les sels des hémi-esters de l'acide carbonique, les sels d'acide carboxylique, les amidines, les guanidines et les composés N-hétérocycliques aromatiques.

2. Procédé selon la revendication 1, **caractérisé en ce que** la base est un composé choisi dans le groupe formé par les carbonates de métal alcalin, les carbonates d'ammonium quaternaire, les carbonates d'alkylammonium quaternaire, la triéthylamine, la N-éthyl-diiso-propylamine, la tri-n-butylamine et la diméthylcyclohexylamine.

3. Procédé selon la revendication 1, **caractérisé en ce que** la base est le carbonate de sodium ou le 1,3-diméthyl-imidazolium-4-carboxylate.

4. Procédé selon les revendications 1 à 3, **caractérisé en ce que** l'on choisit un rapport molaire entre l'acide cyanhydrique et l'ester d'acide mono-chloro-acétique compris entre 1:1 et 1:4.

5. Procédé selon les revendications 1 à 4, **caractérisé en ce que** l'on réalise la réaction à une température comprise entre -25°C et 80°C.

6. Procédé selon les revendications 1 à 5, **caractérisé en ce que** l'on réalise la réaction en présence de la base que l'on met en oeuvre à raison de 75% à 150% en moles par rapport à l'ester mono-chloro-acétique mis en oeuvre.

7. Procédé selon les revendications 1 à 6, **caractérisé en ce que** l'on réalise la réaction en présence d'un catalyseur choisi dans le groupe formé par les éthers bis-(dialkylaminoalkyliques) et les tris-(alcoxyalkyl)amines.
